# EUROPÄISCHE PATENTANMELDUNG

(11) **EP 1 510 186 A1**
(43) Veröffentlichungstag der Anmeldung: **02.03.2005**
(21) Anmeldenummer: 04015713.3
(22) Anmeldetag: 03.07.2004
(51) Int. Cl.: A61C 13/00

(54) **Edelmetall-Keramik-Composites (Cermets) zum Einsatz bei der Herstellung von Zahnersatz**

(30) Priorität: 28.08.2003 DE 10340057
(71) Anmelder: Heraeus Kulzer GmbH, 63450 Hanau (DE)
(72) Erfinder: Brämer, Wulf, Dr., 63486 Bruchköbel (DE); Koops, Ulrich, Dr., 64380 Rossdorf (DE); Dehm, Gerhard, 63579 Freigericht (DE)
(74) Vertreter: Kühn, Hans-Christian

(57) **Zusammenfassung**

Edelmetall-Keramik-Composites (Cermets) werden zum Einsatz bei der Herstellung von Zahnersatz nach CAD/CAM- oder Presskeramik-Verfahren vorgeschlagen.

## Beschreibung

Die Erfindung betrifft den Einsatz von Edelmetall-Keramik-Composites (Cermets) bei der Herstellung von Zahnersatz.

Eines der ältesten Verfahren zur Herstellung von Zahnersatz ist die Metallgusstechnik, bei der ein Wachsmodell der gewünschten Form mit einem feuerfesten Material wie Gips umgeben wird, und flüssiges Metall wie Gold oder dessen Legierungen nach Aushärten die Wachsform ersetzt (Wachs-Ausschmelzverfahren).

Ein weiteres, inzwischen gängiges Verfahren zur Herstellung vor allem von vollkeramischen Dentalrestaurationen ist die sogenannte Presskeramik, bei der dentalkeramisches Material unter Druck und Wärmeeinfluss in einen viskosen Zustand versetzt wird und in eine dem Zahnersatz entsprechende Form hineingedrückt wird. Die Technik und ein dafür geeigneter Pressofen ist z.B. in EP 0 231 773 A1 beschrieben und als Empress® Verfahren bekannt geworden. Das Verfahren eignet sich auch zur Herstellung von Gerüsten für die sogenannte Verblendkeramik. Bei dieser Technik werden in der Regel Metallgerüste mit Keramik so verblendet, dass schließlich zahnfarbener Zahnersatz entsteht.

Neuerdings sind CAD/CAM-Verfahren zur Herstellung von Zahnersatz bekannt geworden (Schmidt A, Walter M, Böning K; CAD/CAM/CIM-Systeme in der restaurativen Zahnheilkunde. Quintessenz 1998,11:1111-1122). Dabei werden in der Regel Keramikrohlinge nach 3D-Datensätzen der angestrebten Dentalrestauration in die gewünschte Form gefräst (EP 904 743, DE 198 38 238 A1, DE 101 07 451A1, US 6,354,836, CEREC®-System von Sirona, PROCERA® -System von Degussa, LAVA® -System von 3M-Espe, Cicero® -System von Elephant Edefmetaal).

Es wurde in DE 36 28 997 C2 vorgeschlagen, im Rahmen des Wachs-Ausschmelzverfahrens sogenannte Cermet-Werkstoffe für verblendbaren Zahnersatz einzusetzen. Cermet-Werkstoffe oder Cermets sind Verbundwerkstoffe aus Metall und Keramik, bevorzugt Oxidkeramik, die pulvermetallurgisch hergestellt werden. DE 36 28 997 C2 betrifft besonders Legierungen aus 50% bis 95% Gold, Rest Platin, Palladium, Silber Indium und/oder Zinn, im Gemisch mit 10% Metalloxid aus der Gruppe Titan-Zirkonium-Zinn- oder Wismutoxid. Als besonderer Vorteil der Cermets wird der geringere Schrumpf bei mit Keramik vergleichbarer Festigkeit dargestellt.

Es wurde nun überraschenderweise gefunden, dass sich solche Cermet-Werkstoffe auf der Basis von Edelmetallen sehr gut für die oben beschriebenen CAD/CAM- oder Presskeramik-Verfahren eignen. Die Erfindung betrifft somit den Einsatz von Cermet-Werkstoffen zur Herstellung von Zahnersatz nach einem CAD/CAM- oder Presskeramik-Verfahren, besonders zur Herstellung von Gerüsten für die Verblendkeramik.

Geeignete Cermet-Werkstoffe haben folgenden Zusammensetzungen: Oxidkeramik mit einem duktilen, biokompatiblen Metall, z.B. Gold, Silber, Platin, Palladium oder Legierungen davon mit gegebenenfalls weiteren Legierungsmetallen, vorzugsweise, Zinn, Zink und/oder Indium. Besonders geeignet sind Gold oder Legierungen mit einem Goldanteil von über 80%.

Der Vorteil des Materials bei der Verwendung als Gerüstmaterial ist, dass es im Gegensatz zu den herkömmlicherweise in der CAD-CAM-Technik verwandten Keramikmaterialien eine bessere Elastizität aufweist. Die Duktilität des Metallanteils bewirkt Dämpfungseigenschaften, so dass Stöße auf das Material nicht so direkt weitergegeben werden wie bei Keramik-Werkstoffen. Der Kauapparat des Zahnersatzträgers wird weniger stark belastet.

Die Materialien eignen sich ferner besonders aufgrund folgender Eigenschaften: Die mechanische Bearbeitung der vollgesinterten Körper ist einfach. Die Härte ist im Vergleich zu vollkeramischen Materialien auf der Basis von Al₂O₃ oder ZrO₂ geringer. Das Material ist duktiler und weniger spröde; es kann einfach und mit weniger anspruchsvollem Werkzeug bearbeitet werden. Ein Nachsintern ist nicht erforderlich. Ein weiterer Vorteil ist, dass die Materialien mit marktgängiger Verblendkeramik bebrannt werden können.

Als Keramik kommen in den Cermets vor allem bekannte Oxidkeramikzusammensetzungen zum Einsatz. Im Gegensatz zu konventionellen Dentalkeramiken sind Oxidkeramiken einphasige Materialien ohne nennenswerte Silikatanteile. Vertreter dieser Werkstoffgruppe, die aufgrund ihrer mechanischen Eigenschaften zu den technischen Hochleistungskeramiken gezählt werden, sind Al₂O₃, MgO-, ZrO₂-, MgAl₂O₄ (Spinell)- und TiO₂-Keramiken. Diese Oxide werden auch zur Optimierung der Festigkeit in andere keramische Massen eingebracht (oxidverstärkte Keramik)¹.
¹*Hahn, R., Wolf, M., Breunig, A.:* Hochleistungskeramik - eine aktuelle Standortbestimmung. Phillip J 13, 311-320 (1996); *Kappert, H.F.:* Keramik als zahnärztlicher Werkstoff. In: Strub, J.R., Türp, J.C., Wit- kowski, S., Hürzeler, M.B, Kern, M. (Hrsg.): Curriculum Prothetik. Bd. II. Quintessenz Verlag, Berlin 1999

Bevorzugt sind Keramiken auf Al₂O₃ oder ZrO₂-Basis.

Die Cermet-Werkstoffe werden nach an sich bekannten Verfahren hergestellt. In der Regel sind das pulvermetallurgische Techniken, die dem Fachmann bekannt sind.

Zweckmäßig liegt der Goldgehalt der in den Cermets eingesetzten Legierungen über 50, bevorzugt über 70% , insbesondere über 80%; der Goldgehalt kann bis an die 100% betragen, aber auch durch die Anwesenheit weiterer Legierungsbestandteile wie Ag, Pt, Pd, Zn, Sn oder In niedriger liegen.

Die in Betracht gezogenen Cermet-Werkstoffe sollten sich möglichst für die Verblendung mit konventionellen Verblendmaterialien eignen. Demnach liegt der Wärmeausdehnungskoeffizient (TEC) zweckmäßig zwischen etwa 13.2 und etwa 15.2 bei 500°C.

Beispiele geeigneter Cermet-Werkstoffe sind folgende vier Zusammensetzungen (Maßangaben in Gew.%):

| | |
|---|---|
| 90% Metall aus | Gold 95%, Indium 3%, Zinn 1 % oder |
| 90% Metall aus | Gold 50%, Platin 35%, Palladium 15% |
| 10% Keramik aus | ZrO₂ oder |
| 10% Keramik | Al₂O₃. |

## Patentansprüche

1. Verwendung eines Cermet-Werkstoffs auf Edelmetall-Basis zur Herstellung von Zahnersatz mit CAD/CAM- oder Presskeramik-Verfahren.

2. Verwendung eines Cermet-Werkstoffs auf Edelmetall-Basis nach Anspruch 1 zur Herstellung von Gerüsten für die Verblendkeramik.

3. Verwendung nach einem der Ansprüche 1 bis 2, wobei der Cermet-Werkstoff auf Edelmetall-Basis im wesentlichen aus einer Oxidkeramik und einem Metall aus der Gruppe Gold, Silber, Platin, Palladium oder Mischungen dieser Metalle oder Legierungen auf Basis dieser Metalle besteht.

4. Verwendung nach einem der Ansprüche 1 bis 3, **dadurch gekennzeichnet, dass** die Oxidkeramik im wesentlichen aus Al₂O₃ oder ZrO₂ oder Mischungen daraus besteht.

5. Verwendung nach Anspruch 3 oder 4, **dadurch gekennzeichnet, dass** die Legierungen eines oder mehrere der Metalle Zn, Sn und In enthalten.

6. Verwendung nach einem der Ansprüche 1 bis 5, **dadurch gekennzeichnet, dass** der Metallanteil des Cermetwerkstoffs zu 50 bis 100% aus Gold besteht.

7. Verwendung nach Anspruch 6, **dadurch gekennzeichnet, dass** der Metallanteil des Cermets zu 80 bis 100% aus Gold besteht.
